# EUROPEAN PATENT APPLICATION

(11) **EP 1 709 879 A2**
(43) Date of publication of application: **11.10.2006**
(21) Application number: 06007133.9
(22) Date of filing: 04.04.2006
(51) Int. Cl.: A41D 19/00, A61B 19/04

(54) **Disposable gloves**

(30) Priority: 04.04.2005 US 667943 P
(71) Applicant: Medline Industries, Inc.,, Mundelein, IL 60060 (US)
(72) Inventor: Weiss, Alan E., Highland Park IL 60035 (US); Bottcher, Paul L., Lakemore IL 60051 (US)
(74) Representative: Grünecker, Kinkeldey, Stockmair & Schwanhäusser Anwaltssozietät

(57) **Abstract**

A disposable glove for a hand of a human wearer that comprises a blend of polyisoprene and another non-latex glove material. The glove provides a fit and feel comparable to a latex glove, while maintaining cost-effectiveness.

## Description

### CROSS-REFERENCE TO RELATED APPLICATION

This application claims the benefit of U.S. Provisional Application No. 60/667,943, filed April 4, 2005, which is hereby incorporated by reference in its entirety.

### FIELD OF THE INVENTION

The present invention relates to gloves and, more specifically, to disposable gloves comprising polyisoprene and other non-latex glove materials.

### BACKGROUND OF THE INVENTION

Disposable gloves are widely used as a protective measure and have become mandatory in many industries and nearly all medical settings. They protect the person wearing the gloves from various objects or materials handled or touched by that person. To allow ease of handling, disposable gloves are made of thin and elastic material to minimize the space between the skin and the glove. One material that has previously enjoyed high usage in manufacturing disposable gloves is latex. Latex is produced from natural rubber trees and is processed to make various products. But many life threatening problems have been associated with the use of latex, including anaphylactic shock. The risk for encountering such problems is especially high in situations involving repeated frequent exposure, such as medical practitioners wearing latex gloves. Latex contains proteins, which may contain allergens that affect a certain percentage of the population. Additionally, the increasing amounts of time that latex gloves are worn has resulted in increased occurrences of adverse symptoms.

Synthetic, non-latex gloves have become the preferred substitute to avoid long-term exposure to allergens associated with latex. Of the currently available non-latex glove materials, vinyl, nitrile, polyurethane, and neoprene (polychloroprene) are available at a relatively low cost, but they do not have many of the desirable characteristics of latex. In particular, these materials are undesirably stiff and, thus, when used alone, they fail to successfully mimic the fit and feel of latex. In contrast, polyisoprene offers a fit, feel, and function almost identical to latex. Polyisoprene, however, is in considerably short supply and is, thus, available at a higher cost.

Therefore, there exists a need to blend polyisoprene with other non-latex glove materials to create a glove that has similar characteristics to a pure polyisoprene or latex glove but which can be made from a reduced weight percent of polyisoprene.

### SUMMARY OF THE INVENTION

According to one embodiment of the present invention, a disposable glove comprises a blend of polyisoprene and at least one additional non-latex glove material. Such non-latex glove material may include vinyl, nitrile, polyurethane, neoprene, or a combination thereof. The ratio of polyisoprene to the at least one additional non-latex glove material may range from about 0.05 to about 0.95. The glove may further comprise a plasticizer, an elastomer, a viscosity reducer, and/or a stabilizing agent. In another embodiment, the glove may also comprise an interior moisturizing layer, therapeutic layer, or moisturizing-therapeutic layer.

According to another embodiment of the present invention, a disposable glove comprises a first polyisoprene layer with a second layer which comprises at least one additional non-latex glove material. The polyisoprene material in the first polyisoprene layer may be comprised of pure polyisoprene or a blend of polyisoprene and at least one additional non-latex glove material. The non-latex glove material may include vinyl, nitrile, polyurethane, neoprene, or a combination thereof. The glove may further comprise a plasticizer, an elastomer, a viscosity reducer, and/or a stabilizing agent. In another embodiment, the glove may also comprise an interior moisturizing layer, therapeutic layer, or moisturizing-therapeutic layer.

According to one method of the present invention, a disposable glove may be formed by providing a first polyisoprene resin and second resin. The second resin comprises at least one additional non-latex glove material. The first resin and the second resin are mixed to form a mixture. A glove-forming surface is dipped into the mixture and dried to form the glove. In one embodiment, the ratio of polyisoprene to the at least one additional non-latex glove material may range from about 0.05 to about 0.95. The non-latex glove material may include vinyl, nitrile, polyurethane, neoprene, or a combination thereof. A plasticizer, an elastomer, a viscosity reducer and/or a stabilizing agent may also be provided and mixed with the first resin, the second resin, or both the first and the second resin to form the mixture. In another embodiment, a moisturizing layer, therapeutic layer, or moisturizing-therapeutic layer may be added to the glove. In another embodiment, the glove-forming surface may be dipped into the mixture a second time and dried in order to increase the thickness of the glove.

According to another method of the present invention, a disposable glove may be formed by providing a first polyisoprene resin and a second resin. The second resin comprises at least one additional non-latex glove material. A glove-forming surface is dipped into the first resin and is subsequently removed from the first resin. The glove-forming surface is then dipped into the second resin and dried on the glove-forming surface to form a coating on the glove. The polyisoprene material may be comprised of pure polyisoprene or a blend of polyisoprene and at least one additional non-latex glove material. The non-latex glove material may include vinyl, nitrile, polyurethane, neoprene, or a combination thereof. A plasticizer, an elastomer, a viscosity reducer, and/or a stabilizing agent may be provided and mixed with the first resin, the second resin, or both the first resin and the second resin. In another embodiment, a moisturizing layer, therapeutic layer, or moisturizing-therapeutic layer may be added to the glove.

The above summary of the present invention is not intended to represent each embodiment or every aspect of the present invention. The detailed description and Figures will describe many of the embodiments and aspects of the present invention.

### BRIEF DESCRIPTION OF THE DRAWINGS

FIG. 1 illustrates a non-latex glove according to one embodiment.

FIG. 2 illustrates a cross-section generally taken through section line 2-2 of the glove shown in FIG. 1.

FIG. 3 illustrates a diagramatic representation of the apparatus used to carry out a method of making the disposable glove of FIG. 1, according to one embodiment.

FIG. 4a illustrates a non-latex glove according to another embodiment.

FIG. 4b illustrates a cross-section generally taken through line 4-4 of the glove shown in FIG. 4a.

FIG. 5 illustrates a diagramatic representation of the apparatus used to carry out a method of making the disposable glove of FIG. 4a, according to another embodiment.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the spirit and scope of the invention.

### DESCRIPTION OF ILLUSTRATIVE EMBODIMENTS

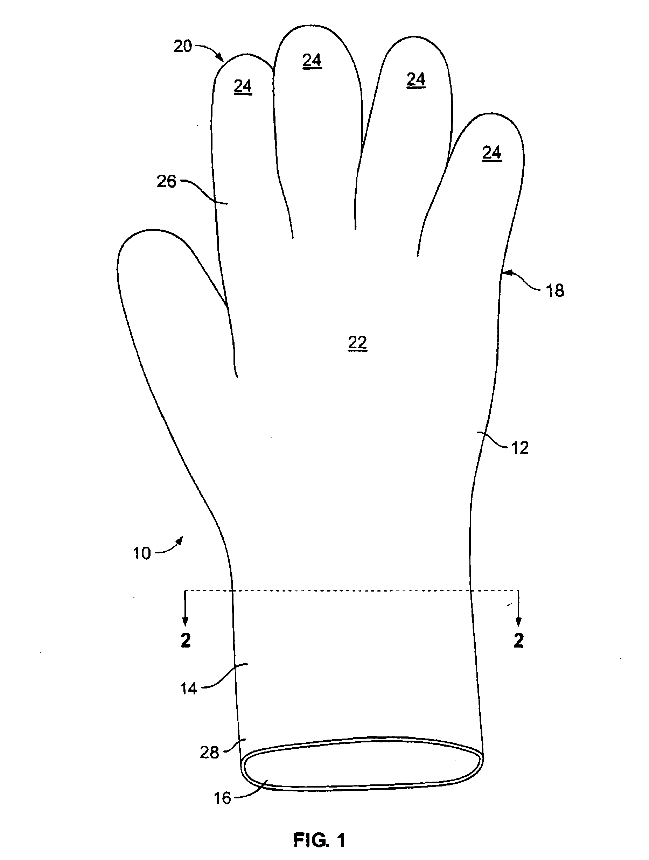

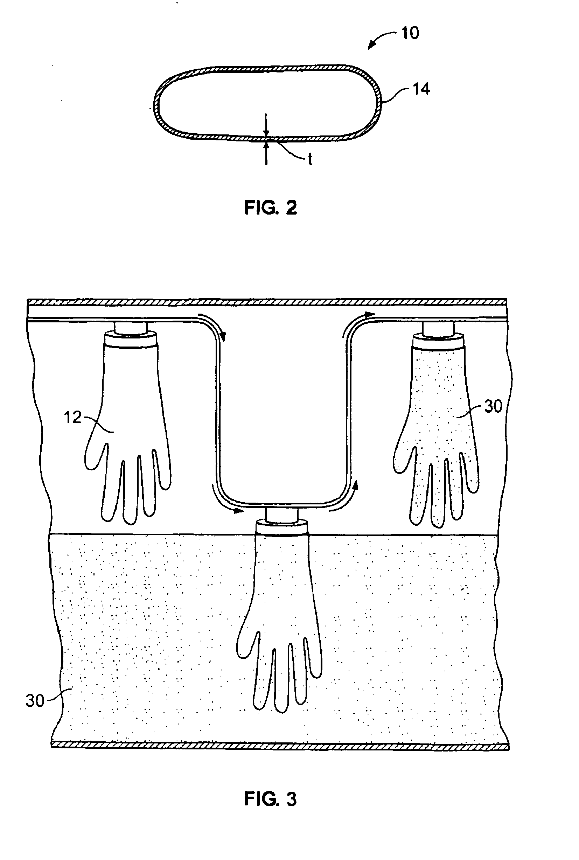

FIG. 1 illustrates a single layer glove 10 comprising a hand area 12 and a wrist area 14 according to one embodiment. The glove 10 of FIG. 1 is typically disposable and substantially impermeable to liquid water. FIG. 2 is a cross-sectional view of the wrist area 14 of the glove 10 taken generally along section line 2-2, shown in FIG. 1, illustrating a generally uniform thickness t.

Still referring to FIG. 1, the glove 10 has an open end 16 into which a hand may be inserted. Opposite the open end 16 is a distal end 18 that is closed to form a closed end 20. The distal end 18 comprises a primary portion 22 and one or more secondary portions 24 located closer to the closed end 20 than the primary portion 22. In one embodiment, the secondary portion 24 terminates at the closed end 20 and defines a chamber smaller than a chamber defined by the primary portion 22. In the illustrated embodiment of FIG. 1, the primary portion 22 includes the hand area 12, whereas one of the secondary portions 24 includes a finger area 26. Similarly, an open-end portion 28 comprises the wrist area 14.

The glove 10 according to one embodiment comprises a blend of polyisoprene and at least one additional non-latex glove material. Suitable examples of non-latex glove materials include, but are not limited to, vinyl, nitrile, polyurethane, neoprene (polychloroprene), and/or combinations thereof. The ratio of polyisoprene to the at least one additional non-latex material includes any composition suitable for creating the fit and feel of latex, ranging from about 0.05 to about 0.95. It is also contemplated that the ratio of polyisoprene to the at least one additional non-latex material may range from about 0.30 to about 0.70. One non-limiting example of a glove according to one embodiment of the present invention includes approximately 50% polyisoprene and approximately 50% neoprene. When a higher ratio of polyisoprene is used, the glove will exhibit characteristics more similar to those of latex; however, the cost of manufacturing these gloves will be substantially higher. Thus, the desired glove composition will vary depending on the desired physical characteristics of the glove, the economic constraints associated with manufacturing the glove, and the availability of polyisoprene. The glove 10 in this embodiment may also comprise a plasticizer, an elastomer, a viscosity reducer, a stabilizing agent, a color pigment, a moisturizing agent, a therapeutic agent, a moisturizing-therapeutic agent, and/or combinations thereof.

According to one embodiment, the glove 10 further comprises a plasticizer. It is contemplated that a number of plasticizers may be used, including those known to one skilled in the art. One example of a desired plasticizer to be used in forming the disposable gloves is diisononyl phthalate (DINP). DINP is desirable because of its balance of several properties. It is contemplated that other plasticizers such as dioctyl phthalate (DOP), diisodecyl phthalate (DIDP), di(2-ethylhexyl) phthalate (DEHP), and/or combinations thereof may be used. It is also contemplated that DINP may be used in combination with DOP, DEHP, and/or DIDP. Table 1 provides a comparative list of several properties associated with DINP, DOP, and DIDP, which are generally referred to as phthalate plasticizers. It is also contemplated that other plasticizers may be used, such as diethylhexyl adipate (DOA), alone or in combination with other plasticizers such as those mentioned above.

**TABLE 1 Phthalate Property Comparison**

| Phthalate | Ester Value | Volume Resistivity OHM-cm, 30°C | Viscosity cps, 25°C | Tensile Strength | Modulus 50% kg/cm² | Molecular Weight |
|---|---|---|---|---|---|---|
| DOP | 287 | 2.0 x 10³ | 54 | 225 | 55 | 391 |
| DINP | 268 | 2.0 x 10³ | 59 | 237 | 61 | 419 |
| DIDP | 251 | 5.0 x 10³ | 72 | 238 | 72 | 447 |

| | | | | | | |
|---|---|---|---|---|---|---|
| Mixture: PVC 100, Plasticizer 50PHR, Stabilizer 1 PHR | | | | | | |

According to one embodiment, the glove 10 further comprises an elastomer. One example of an elastomer that may be used is marketed under the name OLICIZER-20N by Aekyung Petrochemical Co., Ltd. This elastomer has a pH of from about 6.5 to about 7.5, a viscosity of from about 2,000 to 3,000 cps at 25°C. The molecular weight of this elastomer is from about 3,000 to 4,000, and it has a specific gravity of about 1.10 at 25°C.

It is contemplated that a viscosity reducer, a stabilizing agent, and/or a color pigment may be added in forming the glove. For example, in one embodiment, the viscosity reducer is TXIB: 2,2-dimethyl-1-(methylethyl)-1,3-propanediyl bis(2-methylpropanoate). It is contemplated that other viscosity reducers may be used. It is contemplated that stabilizing agents known to those skilled in the art may be used. One example is a preparation containing calcium and zinc soap. Other suitable examples of stabilizing agents include, but are not limited to, xanthan gum, diutan gum, welan gum, gellan gum, carrageenan, pectin, acrylic, copolymer, and polyurethane. Other examples of stabilizing agents that may be used are marketed under the names Kelzan, manufactured by CP Kelco, Inc. (Atlanta, Georgia), and Acrysol SCT-275, manufactured by Rohm & Haas Co. (Philadelphia, Pennsylvania). Color pigment known to those skilled in the art may be added to provide a desired color to the glove. For example, suitable types of color pigments include, but are not limited to, magnesium ferrite, red iron oxide, titanium oxide, yellow iron oxide, phthalocyanine, and prussian green. Other examples of color pigments that may be used are marketed under the names Aquamix 124, manufactured by PolyOne Corp. (Avon Lake, Ohio), and Pigment Red 206, manufactured by Ciba Specialty Chemicals (Basel, Switzerland). It is further contemplated that a moisturizing agent, a therapeutic agent, a moisturizing-therapeutic agent, and/or combinations thereof may be added to the glove. Examples of suitable moisturizing and/or therapeutic agents include water, Aloe Vera, allantoin, alpha hydroxy acids, glycerine, dimethicone, gluconolactone, provitamin B5/Panthenol, and/or Chitosan/Chitin.

Turning to FIG. 3, an apparatus used to carry out a method of making the disposable gloves 10 of FIG. 1 described above is shown according to one embodiment of the present invention. Polyisoprene resin is mixed with a resin of at least one additional non-latex glove material to form a bath of polyisoprene blend material 30. A glove-forming surface shaped like a hand is dipped into the bath of polyisoprene blend material 30. The glove-forming surface, having the polyisoprene blend material thereon, may then be dried, forming the disposable glove 10 having a hand area 12 and a wrist/forearm area 14. The ratio of polyisoprene in the blend includes any percent composition that is suitable to satisfy, for example, the desired glove physical characteristics, the economic constraints associated with manufacturing the glove, and the availability of polyisoprene. It is contemplated that the ratio of polyisoprene in the blend range from about 0.05 to about 0.95. The ratio of polyisoprene in the blend may also may range from about 0.30 to about 0.70. More specifically, the ratio of polyisoprene in the blend may range from about 0.40 to about 0.60. For example, the ratio of polyisoprene in the blend may be approximately 0.50. The disposable glove 10 is then stripped off of the production line, sorted, cleaned/washed, processed, packed, inspected, and sterilized.

In another embodiment, after the glove has been dipped in the bath of polyisoprene blend material 30, it may be dipped into the bath of polyisoprene blend material 30 a second time to obtain a desired glove thickness. Alternatively, the glove may be dipped in a second bath of polyisoprene blend material to obtain a desired glove thickness. The desired thickness of a disposable glove may vary depending on the surgical procedure being performed. For instance, gloves used in eye procedures are generally thinner to allow for greater sensitivity for the surgeon. Gloves used in orthopedic procedures are required to be thicker because of the wear and tear the glove may take. Most gloves range from about 5.5 Mil in thickness (single wall) to about 13 Mil in thickness (single wall). The minimum thickness for a surgical glove is required to be about 4 Mil (single wall) per FDA regulations as defined by ASTM standards.

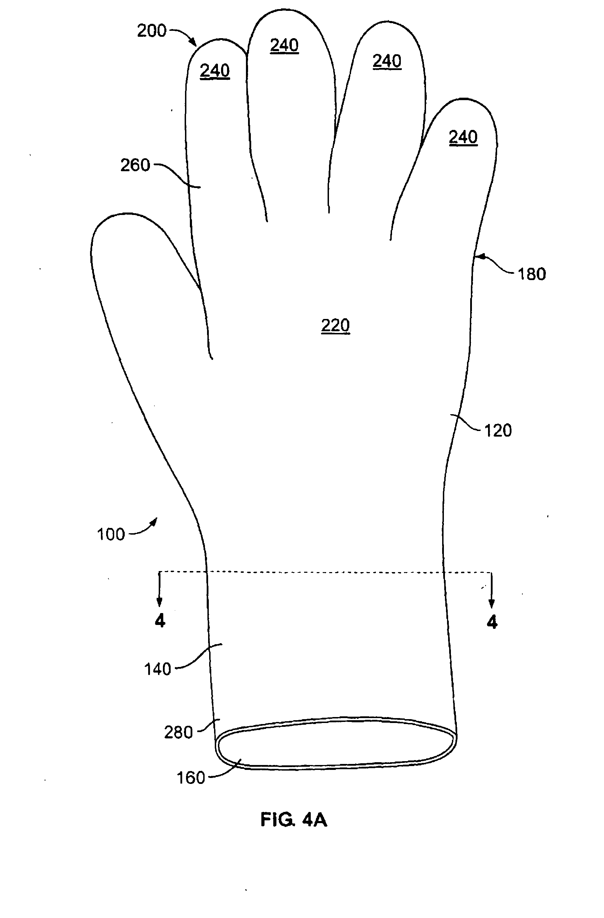

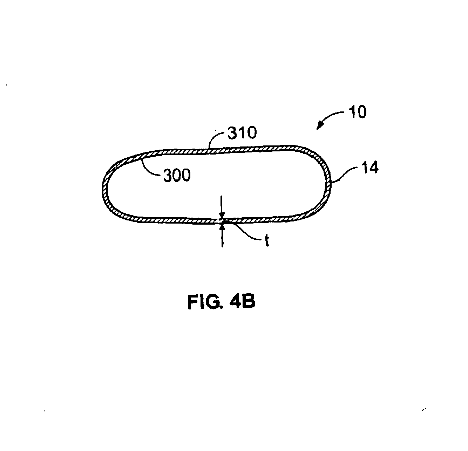

Referring now to FIG. 4a, a disposable glove 100 is shown according to another embodiment. The glove 100 has the same general shape as the glove of FIG. 1 (i.e., a hand area 120, a wrist/forearm area 140, an open end 160, a distal end 180, a closed end 200, a primary portion 220, a secondary portion 240, a finger area 260, and an open-end portion 280). The glove in this embodiment, however, contains two distinct layers: an interior layer 300 and an exterior layer 310. The exterior layer 310 may comprise a different ratio of polyisoprene than the interior layer 300. FIG. 4b shows a cross-sectional view of the glove 100 generally taken along section line 4-4 shown in FIG. 4a. The interior layer 300 comprises polyisoprene. According to one embodiment, the interior layer 300 may be comprised either of pure polyisoprene (i.e., 100 percent polyisoprene or polyisoprene containing small amounts of impurities) or a blend of polyisoprene and at least one additional non-latex glove material. Suitable examples of non-latex glove materials include, but are not limited to, vinyl, nitrile, polyurethane, neoprene, styrene-butadiene-styrene (SBS), styreneisoprene-styrene (SIS), polybutadiene, polyacrylate, variations of butadiene, and/or combinations thereof. One suitable variant of butadiene is manufactured as BP2000 by Dow Reichhold (Research Triangle Park, North Carolina). The exterior layer 310 comprises at least one additional non-latex glove material. According to another embodiment (not shown), the structure of the glove may be reversed, so that the interior layer comprises the at least one additional non-latex glove material, while the exterior layer comprises the polyisoprene material. The glove 100 in either embodiment may also comprise a plasticizer, an elastomer, a viscosity reducer, a stabilizing agent, a color pigment, a moisturizing agent, a therapeutic agent, a moisturizing-therapeutic agent, and/or combinations thereof.

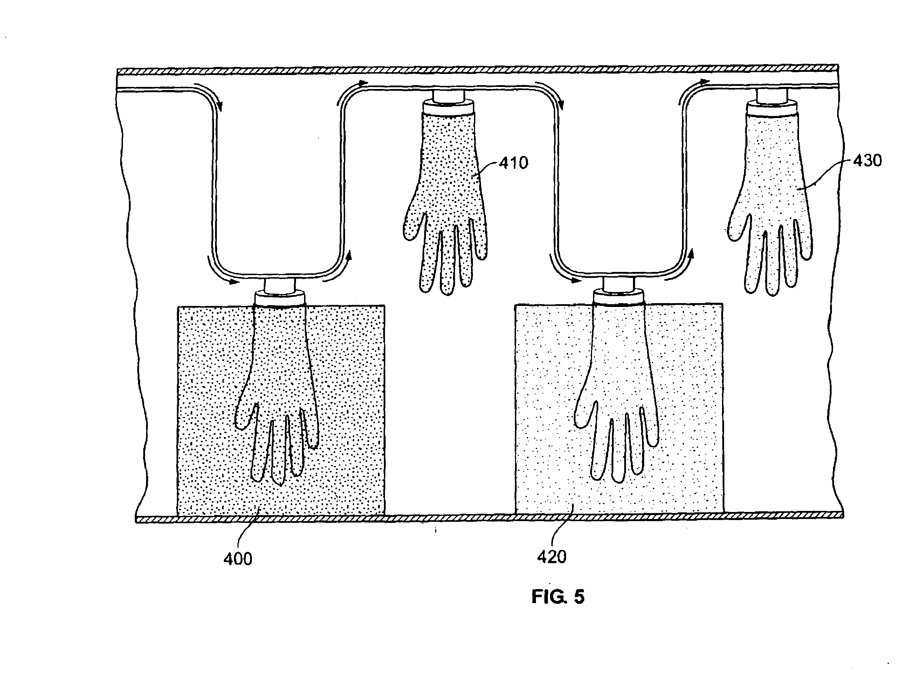

Turning now to FIG. 5, an apparatus used to make the disposable glove 100 of FIG. 4a described above is shown according to one embodiment. A glove-forming surface shaped like a hand is first dipped into a first bath 400. The first bath 400 may comprise a resin of at least one non-latex glove material. Suitable examples of non-latex glove materials include, but are not limited to, vinyl, nitrile, polyurethane, neoprene, and/or combinations thereof. The glove-forming surface is then removed from the first bath 400 and subsequently dipped into a second bath 420 comprising a polyisoprene resin to produce a polyisoprene coating 430. The polyisoprene resin may comprise generally pure polyisoprene or a blend of polyisoprene and at least one additional non-latex glove material. After the glove-forming surface is dipped in the polyisoprene resin 420 as shown in FIG. 5, the glove-forming surface is removed from the polyisoprene resin 420 and dried to form a resulting glove 100. The glove 100 is inverted so that the polyisoprene coating 430 is on the interior surface of the disposable glove 100 as depicted in FIG 4a. The disposable glove 100 is then stripped off of the production line, sorted, cleaned/washed, processed, packed, inspected, and sterilized. The glove 100 in this embodiment may also comprise a plasticizer, an elastomer, a viscosity reducer, a stabilizing agent, a color pigment, a moisturizing agent, a therapeutic agent, a moisturizing-therapeutic agent, and/or combinations thereof.

In an alternative embodiment (not shown), the process is performed by first dipping the glove-forming surface into the polyisoprene resin, and then dipping the glove into the resin of the at least one additional non-latex glove material to form a coating. Using the alternative embodiment, a glove having an interior layer made of at least one additional non-latex glove material and an exterior layer made of polyisoprene is produced. In this embodiment, the polyisoprene resin may comprise generally pure polyisoprene or a blend of polyisoprene and at least one additional non-latex glove material. The glove formed using this embodiment may also comprise a plasticizer, an elastomer, a viscosity reducer, a stabilizing agent, a color pigment, a moisturizing agent, a therapeutic agent, a moisturizing-therapeutic agent, and/or combinations thereof.

The disposable glove described in the embodiments herein may further comprise a coating having moisturizing and/or therapeutic qualities on the interior surface of the glove. Once the glove has been formed on the glove-forming surface by any of the methods described above, the glove-forming surface may be dipped in a final resin (not shown) to produce a moisturizing coating, a therapeutic coating, a moisturizing-therapeutic coating, and/or combinations thereof. Examples of suitable moisturizing coatings, therapeutic coatings, and/or moisturizing-therapeutic coatings include water, Aloe Vera, allantoin, alpha hydroxy acids, glycerine, dimethicone, gluconolactone, provitamin B5/Panthenol, and/or Chitosan/Chitin. After the glove has been dipped in the final resin, the glove may be turned inside out so that the moisturizing coating, the therapeutic coating, and/or the moisturizing-therapeutic coating will be the interior surface in normal use. Alternatively, this coating may be applied to the glove by spraying, dripping, washing, or any suitable technique for applying coatings known in the art. The liquid used to coat the gloves should be generally free of any ingredients that have a deleterious effect on the strength or other properties of the base material of the glove or the anticipated shelf life of the gloves. Examples of suitable liquids for use in coating the gloves include water, Aloe Vera, allantoin, and/or alpha hydroxy acids.

Some desirable properties of a glove include a tensile strength before aging of greater than 10 MPa and typically greater than about 12 MPa for a sample thickness of from about 8 Mil to about 12 Mil as measured in accordance with ASTM D 412-98a. It is more desirable to have a tensile strength before aging of greater than 10 MPa and typically greater than about 13 MPa or about 14 MPa for a sample thickness of from about 8 Mil to about 12 Mil as measured in accordance with ASTM D 412-98a.

The elongation break of a glove is generally greater than 400% and typically greater than 450% or 500% for a sample thickness at the hand area of from about 8 Mil to about 12 Mil as measured in accordance with ASTM D 412-98a. The elongation break of a glove may even be greater than 525% or 550% for a sample thickness at the hand area of from about 8 Mil to about 12 Mil as measured in accordance with ASTM D 412-98a.

It is also desirable to have a 100% stress at a definite elongation of less than 4.5 MPa for a sample thickness at the hand area of from about 8 Mil to about 12 Mil as measured in accordance with ASTM D 412-98a. It is also desirable to have a 100% stress at a definite elongation of less than 4.5 MPa or less than 3.5 MPa for a sample thickness at the hand area of from about 8 Mil to about 12 Mil as measured in accordance with ASTM D 412-98a.

It is also desirable to have a 100% load at a definite elongation of less than 2.8 N for a sample thickness at the hand area of from about 8 Mil to about 12 Mil as measured in accordance with ASTM D 412-98a. It is also desirable to have a 100% load at a definite elongation of less than 2.5 N or less than 2.25 N for a sample thickness at the hand area of from about 8 Mil to about 12 Mil as measured in accordance with ASTM D 412-98a.

It is also desirable to have a glove with a stress remaining of less than 90% of the initial stress. The disposable glove of the present invention has elastic properties such that when the material is stretched from an initial configuration to fit about an object such as a hand, the material conforms to the object. The material initially exerts an initial pressure on the object and thereafter relaxes to exert a reduced pressure on the object, which is substantially less than about 90% of the initial pressure. This reduction in pressure is generally reached within minutes after the material is stretched to fit around the object. Thus, the gloves are particularly useful as surgical gloves because they do not substantially restrict movement or blood flow of the wearer's fingers, and, thus, the gloves do not cause fatigue or numbness.

### Example 1

Laboratory testing of a glove comprising approximately 50% polyisoprene and approximately 50% neoprene was performed. The 50% polyisoprene, 50% neoprene glove was tested using an Instron® Universal Testing System (Burlington, Ontario), which measured the tensile strength (Tₛ), elongation (E_{b}) and modulus (M500) as determined by ASTM D 412-98a, ASTM D 624-00e1, and ASTM D 1894-00. The 50% polyisoprene, 50% neoprene glove was then aged in an oven maintained at a temperature of about 70°C for approximately 168 hours to determine the effects of the aging on the glove.

Similar testing was performed on comparative gloves comprising approximately 100% polyisoprene and 100% latex. The properties of the 50% polyisoprene, 50% neoprene glove compared to those of the comparative 100% polyisoprene glove and the 100% latex glove are set forth in Table 2 below.

**TABLE 2**

| **Sample** | **Unaged** | | | **Aged 70 °C @ 168 hrs** | | **Thickness (mm)** | | |
|---|---|---|---|---|---|---|---|---|
| | ***Ts (MPa)*** | ***E***_{***b***} ***(%)*** | ***M500 (MPa)*** | ***Ts (MPa)*** | ***E***_{***b***} ***(%)*** | **Cuff** | **Palm** | **Finger** |
| **50% Polyisoprene / 50% Neoprene Blend** | | | | | | 0.17 | 0.22 | 0.24 |
| | | | | | 740 | | | |
| | 17.8 | 810 | 2.15 | 18.2 | | | | |
| **100% Polyisoprene** | | | | | 810 | 0.17 | 0.19 | 0.21 |
| | 22.8 | 870 | 1.52 | 21.2 | | | | |
| **100% Latex** | 29.0 | 850 | 3.2 | 27.0 | 800 | 0.16 | 0.20 | 0.24 |

As shown in Table 2, the 50% polyisoprene, 50% neoprene glove had properties comparable to the 100% polyisoprene glove. For example, the tensile strength of the 50% polyisoprene, 50% neoprene glove was 17.8 MPa, and the tensile strength of the 100% polyisoprene gloves was 22.8 MPa. Other properties of the 50% polyisoprene, 50% neoprene glove also compared similarly to those of the 100% polyisoprene glove. Although the properties of the 100% polyisoprene glove were generally more similar to the 100% latex glove than the 50% polyisoprene, 50% neoprene glove, the 50% polyisoprene, 50% neoprene glove exceeded the appropriate ASTM specification for synthetic gloves.

According to alternative embodiment A, a disposable glove comprises a blend of polyisoprene and at least one additional non-latex glove material.

According to alternative embodiment B, the disposable glove of alternative embodiment A, wherein the at least one additional non-latex glove material comprises vinyl, nitrile, polyurethane, neoprene, or a combination thereof.

According to alternative embodiment C, the disposable glove of alternative embodiment A, wherein the glove comprises a ratio of polyisoprene to at least one additional non-latex glove material of from about 0.05 to about 0.95.

According to alternative embodiment D, the disposable glove of alternative embodiment A, wherein the glove further comprises a plasticizer.

According to alternative embodiment E, the disposable glove of alternative embodiment A, wherein the glove further comprises an elastomer.

According to alternative embodiment F, the disposable glove of alternative embodiment A, wherein the glove further comprises a viscosity reducer.

According to alternative embodiment G, the disposable glove of alternative embodiment A, wherein the glove further comprises a stabilizing agent.

According to alternative embodiment H, the disposable glove of alternative embodiment A, wherein the glove further comprises an interior moisturizing layer, therapeutic layer, or a moisturizing-therapeutic layer.

According to alternative embodiment I, a disposable glove comprises a first layer comprising polyisoprene and a second layer comprising at least one additional non-latex glove material.

According to alternative embodiment J, the disposable glove of alternative embodiment I, wherein the first layer comprises generally pure polyisoprene.

According to alternative embodiment K, the disposable glove of alternative embodiment I, wherein the first layer comprises a blend of polyisoprene and at least one additional non-latex glove material.

According to alternative embodiment L, the disposable glove of alternative embodiment I, wherein the at least one additional non-latex glove material comprises vinyl, nitrile, polyurethane, neoprene, or a combination thereof.

According to alternative embodiment M, the disposable glove of alternative embodiment I, wherein the glove further comprises a plasticizer.

According to alternative embodiment N, the disposable glove of alternative embodiment I, wherein the glove further comprises an elastomer.

According to alternative embodiment O, the disposable glove of alternative embodiment I, wherein the glove further comprises a viscosity reducer.

According to alternative embodiment P, the disposable glove of alternative embodiment I, wherein the glove further comprises a stabilizing agent.

According to alternative embodiment Q, the disposable glove of alternative embodiment I, wherein the glove further comprises an interior moisturizing layer, therapeutic layer, or moisturizing-therapeutic layer.

According to alternative embodiment R, a method of forming a disposable glove comprises the acts of providing a first polyisoprene resin and a second resin, the second resin comprising at least one additional non-latex glove material, mixing the first resin and the second resin to form a mixture, dipping a glove-forming surface into the mixture, and drying the mixture to form the glove.

According to Alternative Embodiment S, the method of alternative embodiment R further comprises providing a plasticizer and mixing the plasticizer with the first polyisoprene resin and the second resin of at the least one additional non-latex glove material to form the mixture.

According to alternative embodiment T, the method of alternative embodiment S, wherein the plasticizer comprises diisononyl phthalate, dioctyl phthalate, diisodecyl phthalate, diisodecyl phthalate, or combinations thereof.

According to alternative embodiment U, the method of alternative embodiment S, wherein the plasticizer is diisononyl phthalate.

According to alternative embodiment V, the method of alternative embodiment R further comprises providing an elastomer and mixing the elastomer with the first polyisoprene resin and the second resin of the at least one additional non-latex glove material to form the mixture.

According to alternative embodiment W, the method of alternative embodiment R further comprises providing a viscosity reducer and mixing the viscosity reducer with the first polyisoprene resin and the second resin of the at least one additional non-latex glove material to form the mixture.

According to alternative embodiment X, the method of alternative embodiment R further comprises providing a stabilizing agent and mixing the stabilizing agent with the first polyisoprene resin and the second resin of the at least one additional non-latex glove material to form the mixture.

According to alternative embodiment Y, the method of alternative embodiment R, wherein the mixture comprises a ratio of polyisoprene to at least one additional non-latex glove material of from about 0.05 to about 0.95.

According to alternative embodiment Z, the method of alternative embodiment R, wherein the at least one additional non-latex glove material comprises vinyl, nitrile, polyurethane, neoprene, or a combination thereof.

According to alternative embodiment AA, the method of alternative embodiment R further comprises dipping the glove into the mixture a second time and drying the glove such that the thickness of the glove is increased.

According to alternative embodiment AB, the method of alternative embodiment R further comprises adding an interior moisturizing layer, therapeutic layer, or moisturizing-therapeutic layer.

According to alternative embodiment AC, a method of forming a disposable glove comprises the acts of providing a first polyisoprene resin and a second resin, the second resin comprising at least one additional non-latex glove material, dipping a glove-forming surface into the first polyisoprene resin, removing the glove-forming surface from the first polyisoprene resin, dipping the glove-forming surface into the second resin, and drying the second resin on the glove-forming surface to form a coating on the glove.

According to alternative embodiment AD, the method of alternative embodiment AC, wherein the first polyisoprene resin is pure polyisoprene.

According to alternative embodiment AE, the method of alternative embodiment AC, wherein the first polyisoprene resin comprises a blend of polyisoprene and at least one additional non-latex glove material.

According to alternative embodiment AF, the method of alternative embodiment AC, wherein the at least one additional non-latex glove material comprises vinyl, nitrile, polyurethane, neoprene, or a combination thereof

According to alternative embodiment AG, the method of alternative embodiment AC further comprises providing a plasticizer and mixing the plasticizer with the first polyisoprene resin, the second resin, or both the first polyisoprene resin and the second resin.

According to alternative embodiment AH, the method of alternative embodiment AG, wherein the plasticizer comprises diisononyl phthalate, dioctyl phthalate, diisodecyl phthalate, diisodecyl phthalate, or combinations thereof.

According to alternative embodiment AI, the method of alternative embodiment AG,wherein the plasticizer is diisononyl phthalate.

According to alternative embodiment AJ, the method of alternative embodiment AC further comprises providing an elastomer and mixing the elastomer with the first polyisoprene resin, the second resin, or both the first polyisoprene resin and the second resin.

According to alternative embodiment AK, the method of alternative embodiment AC further comprises providing a viscosity reducer and mixing the viscosity reducer with the first polyisoprene resin, the second resin, or both the first polyisoprene resin and the second resin.

According to alternative embodiment AL, the method of alternative embodiment AC further comprises providing a stabilizing agent and mixing the stabilizing agent with the first polyisoprene resin, the second resin, or both the first polyisoprene resin and the second resin.

According to alternative embodiment AM, the method of alternative embodiment AC further comprises adding an moisturizing layer, a therapeutic layer, or a moisturizing-therapeutic layer.

While the present invention has been described with reference to one or more particular embodiments, those skilled in the art will recognize that many changes may be made thereto without departing from the spirit and scope of the present invention. Each of these embodiments and obvious variations thereof is contemplated as falling within the spirit and scope of the invention, which is set forth in the following claims.

## Claims

1. A disposable glove comprising a blend of polyisoprene and at least one additional non-latex glove material.

2. The disposable glove of claim 1, wherein the at least one additional non-latex glove material comprises vinyl, nitrile, polyurethane, neoprene, or a combination thereof.

3. The disposable glove of claim 1, wherein the glove comprises a ratio of polyisoprene to at least one additional non-latex glove material of from about 0.05 to about 0.95.

4. The disposable glove of claim 1, wherein the glove further comprises a plasticizer.

5. The disposable glove of claim 1, wherein the glove further comprises an elastomer.

6. The disposable glove of claim 1, wherein the glove further comprises a viscosity reducer.

7. The disposable glove of claim 1, wherein the glove further comprises a stabilizing agent.

8. The disposable glove of claim 1, wherein the glove further comprises an interior moisturizing layer, therapeutic layer, or a moisturizing-therapeutic layer.

9. A disposable glove comprising:
a first layer comprising polyisoprene; and
a second layer comprising at least one additional non-latex glove material.

10. The disposable glove of claim 9, wherein the first layer comprises generally pure polyisoprene.

11. The disposable glove of claim 9, wherein the first layer comprises a blend of polyisoprene and at least one additional non-latex glove material.

12. The disposable glove of claim 9, wherein the at least one additional non-latex glove material comprises vinyl, nitrile, polyurethane, neoprene, or a combination thereof

13. The disposable glove of claim 9, wherein the glove further comprises a plasticizer.

14. The disposable glove of claim 9, wherein the glove further comprises an elastomer.

15. The disposable glove of claim 9, wherein the glove further comprises a viscosity reducer.

16. The disposable glove of claim 9, wherein the glove further comprises a stabilizing agent.

17. The disposable glove of claim 9, wherein the glove further comprises an interior moisturizing layer, therapeutic layer, or moisturizing-therapeutic layer.

18. A method of forming a disposable glove comprising the steps of:
providing a first polyisoprene resin and a second resin, the second resin comprising at least one additional non-latex glove material;
mixing the first resin and the second resin to form a mixture;
dipping a glove-forming surface into the mixture; and
drying the mixture to form the glove.

19. The method of claim 18 further comprising providing a plasticizer and mixing the plasticizer with the first polyisoprene resin and the second resin of at the least one additional non-latex glove material to form the mixture.

20. The method of claim 19, wherein the plasticizer comprises diisononyl phthalate, dioctyl phthalate, diisodecyl phthalate, diisodecyl phthalate, or combinations thereof.

21. The method of claim 19, wherein the plasticizer is diisononyl phthalate.

22. The method of claim 18 further comprising providing an elastomer and mixing the elastomer with the first polyisoprene resin and the second resin of the at least one additional non-latex glove material to form the mixture.

23. The method of claim 18 further comprising providing a viscosity reducer and mixing the viscosity reducer with the first polyisoprene resin and the second resin of the at least one additional non-latex glove material to form the mixture.

24. The method of claim 18 further comprising providing a stabilizing agent and mixing the stabilizing agent with the first polyisoprene resin and the second resin of the at least one additional non-latex glove material to form the mixture.

25. The method of claim 18, wherein the mixture comprises a ratio of polyisoprene to at least one additional non-latex glove material of from about 0.05 to about 0.95.

26. The method of claim 18, wherein the at least one additional non-latex glove material comprises vinyl, nitrile, polyurethane, neoprene, or a combination thereof.

27. The method of claim 18 further comprising dipping the glove into the mixture a second time and drying the glove such that the thickness of the glove is increased.

28. The method of claim 18 further comprising adding an interior moisturizing layer, therapeutic layer, or moisturizing-therapeutic layer.

29. A method of forming a disposable glove comprising the acts of:
providing a first polyisoprene resin and a second resin, the second resin comprising at least one additional non-latex glove material;
dipping a glove-forming surface into the first polyisoprene resin;
removing the glove-forming surface from the first polyisoprene resin;
dipping the glove-forming surface into the second resin; and
drying the second resin on the glove-forming surface to form a coating on the glove.

30. The method of claim 29, wherein the first polyisoprene resin is pure polyisoprene.

31. The method of claim 29, wherein the first polyisoprene resin comprises a blend of polyisoprene and at least one additional non-latex glove material.

32. The method of claim 29, wherein the at least one additional non-latex glove material comprises vinyl, nitrile, polyurethane, neoprene, or a combination thereof.

33. The method of claim 29 further comprising providing a plasticizer and mixing the plasticizer with the first polyisoprene resin, the second resin, or both the first polyisoprene resin and the second resin.

34. The method of claim 33, wherein the plasticizer comprises diisononyl phthalate, dioctyl phthalate, diisodecyl phthalate, diisodecyl phthalate, or combinations thereof.

35. The method of claim 33, wherein the plasticizer is diisononyl phthalate.

36. The method of claim 29 further comprising providing an elastomer and mixing the elastomer with the first polyisoprene resin, the second resin, or both the first polyisoprene resin and the second resin.

37. The method of claim 29 further comprising providing a viscosity reducer and mixing the viscosity reducer with the first polyisoprene resin, the second resin, or both the first polyisoprene resin and the second resin.

38. The method of claim 29 further comprising providing a stabilizing agent and mixing the stabilizing agent with the first polyisoprene resin, the second resin, or both the first polyisoprene resin and the second resin.

39. The method of claim 29 further comprising adding an moisturizing layer, a therapeutic layer, or a moisturizing-therapeutic layer.
